# FASCICULE DE BREVET EUROPEEN

(11) **EP 1 673 479 B1**
(45) Date de publication et mention de la délivrance du brevet: **31.12.2008**
(21) Numéro de dépôt: 04817156.5
(22) Date de dépôt: 08.10.2004
(51) Int. Cl.: C12Q 1/68

(54) **METHODE DE DETECTION RAPIDE DE MICRO-ORGANISMES SUR PUCES A ADN**
VERFAHREN ZUM SCHNELLEN NACHWEIS VON MIKROORGANISMEN AUF DNA-CHIPS
METHOD FOR THE RAPID DETECTION OF MICRO-ORGANISMS ON DNA CHIPS

(30) Priorité: 10.10.2003 FR 0311921
(43) Date de publication de la demande: 28.06.2006
(60) Demande divisionnaire: 08164868.5
(73) Titulaire: Bertin Technologies S.A., 78180 Montigny-le-Bretonneux (FR); CENTRE NATIONAL DE LA RECHERCHE SCIENTIFIQUE (CNRS), 75794 Paris Cedex 16 (FR)
(72) Inventeur: RAVASSARD, Philippe, F-75013 Paris (FR); BIZET, Karine, F-92100 Boulogne Billancourt (FR); MALLET, Jacques, F-75013 Paris (FR); VERDIER, Amandine, F-78000 Versailles (FR)
(74) Mandataire: Ramey, Daniel
(86) Numéro de dépôt international: PCT/FR2004/002558
(87) Numéro de publication internationale: WO 2005/035785

(56) Documents cités:
- EP-A- 0 353 124
- EP-A- 1 447 454
- EP-A- 1 473 370
- WO-A-00/52203
- WO-A-01/36683
- WO-A-01/77372
- WO-A-03/031654
- WO-A-03/083055
- FR-A- 2 801 609
- US-A1- 2003 143 571
- US-A1- 2003 165 824
- TROESCH ET AL: "MYCOBACTERIUM SPECIES IDENTIFICATION AND RIFAMPICIN RESISTANCE TESTING WITH HIGH-DENSITY DNA PROBE ARRAYS" JOURNAL OF CLINICAL MICROBIOLOGY, WASHINGTON, DC, US, vol. 37, no. 1, janvier 1999 (1999-01), pages 49-55, XP002130858 ISSN: 0095-1137
- SMALL JACK ET AL: "Direct detection of 16S rRNA in soil extracts by using oligonucleotide microarrays" APPLIED AND ENVIRONMENTAL MICROBIOLOGY, WASHINGTON,DC, US, vol. 67, no. 10, octobre 2001 (2001-10), pages 4708-4716, XP002245295 ISSN: 0099-2240
- BOSCH J ET AL: "Validation of sequence optimized 70 base pair oligonucleotides for use on DNA microarrays" BIOSIS, vol. 12, no. 88, 12 septembre 2000 (2000-09-12), XP002184313
- "Fiche technique ICN, référence 1002417: Brain heart infusion broth" pages 1-2,
- DIRECTION DES ALIMENTS (SANTE CANADA): "Glossaires des bouillons, géloses et autres réactifs, Section B" janvier 2006 (2006-01), pages 1-2, Extrait de l'Internet: URL:http://www.hc-sc.gc.ca/fn-an/res-rech/ analy-meth/microbio/index_f.html>
- ROSENOW ET AL: "Prokaryotic RNA preparation methods useful for high density array analysis: comparison of two approaches" NUCLEIC ACIDS RESEARCH, vol. 29, no. 22, 2001, page E112,
- WENDISCH ET AL: "Isolation of Escherichia coli mRNA and comparison of expression using mRNA and total RNA on DNA microarrays" ANALYTICAL BIOCHEMISTRY, vol. 290, 2001, pages 205-213,

## Description

La présente invention porte sur une méthode rapide d'analyse d'une population microbienne d'un environnement donné, notamment d'une population microbienne prélevée dans l'air. En particulier, la méthode utilisée comprend l'utilisation de puces à ADN, avec une méthode de détection radioactive ou chimiluminescente.

La majorité des méthodes de détection utilisées aujourd'hui sont fondées sur l'analyse de l'ADN ou de l'ARN et comprennent la mise en oeuvre de la technique d'amplification PCR décrite dans les brevets US 4,683,195 et 4,683,202.

La PCR permet d'amplifier des séquences cibles d'acides nucléiques qui sont ensuite détectées par des méthodes classiques de détection. Cependant, la mise en oeuvre d'une réaction d'amplification requiert environ 2 heures.

D'autres techniques sont décrites dans l'état de la technique, incluant les procédés d'hybridation telles que le Northem Blot ou le Southem Blot, utilisant des sondes, ou encore des techniques en microplaques telles que le test ELISA. Ces techniques sont délicates à mettre en oeuvre et relativement chères. En particulier, un grand nombre de microplaques et un volume d'échantillons très important seraient nécessaires pour l'analyse de plusieurs pathogènes différents en parallèle en microplaques.

Récemment, on a décrit des techniques d'hybridations sur des supports solides appelés puces (US 6,458,584). De telles puces sont par exemple fabriquées par synthèse parallèle d'oligonucléotides (Matson et al., Anal Biochem., 1995, 224, 110-116), fixées à la surface d'un support de verre (Ghu et al., 1994, Nucl. Acids Res. 22, 5456-54.65), de feuilles de polypropylène (Matson *et al.,* supra 1995) ou de gel (Khrapko et al., 1991, J. DNA Sequencing 1 : 375-388).

Un grand nombre d'hybridation peut être réalisé en parallèle en utilisant une petite quantité d'échantillon. Cependant, le temps d'hybridation reste assez long, compris en général entre 16 et 24 heures.

Malgré l'existence de nombreuses méthodes disponibles, aucune ne permet une détection rapide, par exemple inférieure à 10 heures, voire inférieure à 3 heures. Or, dans le cas d'une infection par *Bacillus anthracis,* l'attribution précoce d'un antibiotique est déterminante pour limiter la mortalité. Une détection rapide est par conséquent recherchée dans le cas de contamination par des armes biologiques, afin de sauver le plus grand nombre de vies.

La demande US2003/0165824 décrit une méthode de détection rapide de micro-organismes (Mycobacterium sp.). ("Summary of the invention"; Exemple 6: [0055]-[0058]; [0053]) comprenant: la collecte de micro-organismes et l'extraction de l'ARN des micro-organismes ([0033], [0034]), une étape d'amplification ([0032], [0035]), le marquage de l'ARN et son hybridation à l'aide de sondes de capture placées sur une puce et spécifiques de chaque micro-organisme ([0037], [0057], Tableau 7), caractérisé en ce que l'hybridation est effectuée dans un temps de 45 minutes, la détection de l'hybridation et l'analyse des résultats permettant de déterminer si les micro-organismes sont présents ([0037], [0057], [0058], Tableau 7).

De nombreuses espèces de micro-organismes peuvent se présenter sous forme de spores, qui restent à l'état de dormance en réponse à des stress environnementaux. Le processus durant lequel les bactéries dormantes se transforment en cellules végétatives est connu sous le nom de germination. Parmi les bactéries susceptibles de sporuler, on citera par exemple *Bacillus anthracis, Baccillus cereus, Clostridium botulinum abd Clostridiun perfringens, Vibrio cholera, Vibrio vulnificus, Micrococcus luteus, Micrococcus tuberculosis and Legionella pneumophila.*

Afin de détecter des bactéries présentes dans l'échantillon sous une forme sporulée, leur culture pendant une période d'au moins 24 heures est en général préconisée. Cependant, la mise en culture pour germination des formes sporulées allonge d'autant le temps de détection. En outre, la culture nécessite l'utilisation de conditions stériles.

A la connaissance de la demanderesse, il n'a jamais été décrit dans l'état de la technique une méthode rapide de détection de micro-organismes, suffisamment sensible et spécifique.

Il n'a jamais été décrit de méthode rapide de détection de micro-organismes permettant la détection de spores bactériennes.

Enfin, à la connaissance de la demanderesse, aucune méthode de détection par chimiluminescence sur puce à ADN suffisamment sensible, et évitant une trop forte diffusion de la lumière à la surface de la puce n'a été décrite.

L'invention vise précisément une méthode de détection rapide de micro-organismes d'intérêts dans un échantillons, par hybridation moléculaire sur puces à ADN, des ARN spécifiques de ces organismes. Ces ARN ont été, dans une première étape, rétro-transcrits en ADN complémentaire (ADNc), assurant ainsi le marquage des sondes échantillons et permettant deux modes de détection possibles sur puces à ADN, à savoir un mode radioactif et un mode chimiluminescent. L'ensemble des étapes de la méthode selon l'invention est automatisable et permet à partir de la collecte de l'échantillon d'obtenir un résultat d'identification en 2h30 pour le mode radioactif, et en un peu plus de 3 heures pour le mode chimiluminescent.

Pour la mise au point d'une chaîne globale d'identification rapide, l'invention résulte en particulier d'une réduction très importante du temps d'hybridation. En effet, il est en général conseillé dans la littérature scientifique un temps d'hybridation d'au moins 16 heures. Ce temps a été réduit à 30 minutes en tirant partie des propriétés de cinétique de la réaction d'hybridation.

En effet, la vitesse de réaction permettant la formation des duplex sonde échantillon (ADNc)/sonde de capture (immobilisées sur la puce) dépend principalement de la taille de ces duplex. Ainsi, en utilisant des sondes de captures immobilisées de petite taille (oligonucléotides de 30 à 90 mers, préférablement 70 mers), il a été constaté que le temps d'hybridation pouvait être diminué fortement.

Par conséquent, la présente invention fournit une méthode de détection rapide de micro-organismes, comprenant :
(a) la collecte de micro-organismes,
(b) l'extraction de l'ARN des micro-organismes,
(c) le marquage de l'ARN ou sa conversion en ADN complémentaire marqué,
(d) l'hybridation de l'ADN complémentaire marqué ou de l'ARN marqué obtenu à l'étape (c) à l'aide de sondes de capture, de 30 a 90 mers, placées sur une puce et spécifiques de chaque micro-organisme, caractérisée en ce que l'hybridation est effectuée dans un temps compris entre 15 et 45 minutes, de préférence environ 30 minutes,
(e) la détection de l'hybridation éventuelle, et,
(f) l'analyse des résultats permettant de déterminer si les micro-organismes sont présents.

Est également fournie une méthode pour diminuer la diffusion de la lumière sur une puce pour la détection en mode chimiluminescent, ladite méthode comprenant :
(a) la préparation d'une puce sur la surface de laquelle est déposée une membrane poreuse imprégnée d'un substrat chimiluminescent, permettant ainsi d'immobiliser la phase liquide du substrat.

La puce comprenant la membrane poreuse est également décrite.

Au sens de l'invention, le terme "environnement" inclue l'eau, l'air, les plantes et le sol. Des exemples d'échantillons environnementaux utilisables dans la méthode de l'invention sont par exemple, de l'eau, des eaux usées, le sol, la boue, des déchets, des sédiments, l'air, etc...

Les échantillons incluent des échantillons biologiques prélevés chez l'homme ou l'animal. La méthode convient à tout type d'échantillons biologiques. Des exemples d'échantillons biologiques incluent, sans être limités, le liquide céphalo-rachidien, la lymphe, le liquide synovial, les larmes, l'urine, le sang, le plasma, le sérum, la peau, les tumeurs, les liquides amniotiques, des tissus, des cellules et des cultures cellulaires.

Au sens de l'invention, on entend par "acide nucléique cible", un acide nucléique dérivé d'un échantillon biologique ou environnemental, détecté par la sonde de capture par hybridation spécifique.

Ainsi, l'acide nucléiques cible a une séquence qui est complémentaire de la sonde de capture correspondante et est marquée avec un marqueur radioactif ou chimiluminescent.

Le terme « sonde » fait référence dans le texte qui suit à un acide nucléique capable de cibler un autre acide nucléique par appariement de bases complémentaires, incluant des bases naturelles telles que A, G, C, U ou T ou des bases modifiées telles que l'ionosine par exemple. La sonde de capture est utilisée dans la présente invention pour lier l'acide nucléique cible si une telle cible est présente dans l'échantillon.

Au sens de l'invention, le terme "micro-organismes" inclue toutes bactéries gram-positive ou gram-négative. Les bactéries susceptibles d'être détectées par le procédé de l'invention sont de préférence pathogènes pour les hommes ou les animaux et/ou invasives. Les différents types de bactéries susceptibles d'être détectées par les méthodes de l'invention, incluent *Staphylococcus aureus, Staphylococcus epidermidis, Staphylococcus saprophyticus, Micrococcus luteus, Micrococcus roseus, Lactococcus lactis, Streptococcus pneumoniae, Streptococcus bovis, Leuconostoc mesenteroides, Oenococcus oeni, Pediococcus damnosus, Enterococcus durans, Enterococcus faecalis, Enterococcus faecium, Neisseria gonorrhoeae, Neisseria meningitidis, Moraxella catarrhalis, Acinetobacter baumannii, Acinetobacter Iwoffii, Citrobacter diversus, Citrobacter freundii, Citrobacter diversus, Edwardsiella tarda, Enterobacter cloacae, Erwinia chrysantum, Erwinia carotovora, Escherichia coli, Hafnia alvei, Klebsiella oxytoca, Klebsiella pneumoniae, Morganella morganii, Pantoea agglomerans, Proteus mirabilis, Proteus vulgaris, Providencia alcalifaciens, Salmonella enterica subsp. enterica, Salmonella enterica, Salmonella paratyphiSerratia* marcescens, *Shigella flexneri, Yersinia enterocolitica; Yersinia pestis, Vibrio parahaemolyticus, Vibrio cholerae,* Aeromonas *hydrophila,* Pseudomonas *aeruginosa, Pseudomonas fluorescens, Pseudomonas putida, Burkholderia cepacia, Burkholderia mallei, Burkholderia pseudomallei, Ralstonia pickettii, Alcaligenes faecalis, Acinetobacter baumannii, Acinetobacter Iwoffii, Stenotrophomonas maltophilia, Campylobacter jejuni, Bacillus cereus, Bacillus thuringiensis, Bacillus subtilis, Bacillus anthracis, Bacillus globigii, Listeria monocytogenes, Lactobacillus casei, Clostridium botulinum, Clostridium perfringens, Mycobacterium bovis BCG, Legionella pneumophila, Bordetella pertussis, Francisella tularensis, Brucella* melitensis, *Brucella abortus, Brucella suis, Brucella canisCoxiella burnetti, Chlamydia pneumonia.* Cette liste n'est bien entendu pas limitative.

Plus précisément, la présente invention fournit une méthode de détection rapide des micro-organismes. Les micro-organismes sont collectés par n'importe quel moyen approprié connu.

Pour les échantillons d'airs, des dispositifs de collecte d'air ou des échantillonneurs d'air peuvent être utilisés, comportant des milieux gélosés ou des filtres membranaires poreux ou permettant de récupérer les micro-organismes dans un milieu liquide défini.

Si les échantillons sont sous une forme végétative, ils sont soumis à une étape de lyse et une étape d'extraction comme décrit ci-dessous. Cependant, si les échantillons sont sous forme sporulée, les échantillons sont soumis à une étape de revivification. Cette étape de revivification assure un début de germination des spores et permet de s'affranchir de l'extrême difficulté à réaliser une lyse efficace des formes sporulées. La forme sporulée des bactéries se caractérise par une réduction de l'activité physiologique de la cellule, une déshydratation interne et par la formation d'enveloppes externes extrêmement résistantes. Cette forme confère aux bactéries une grande résistance vis à vis du milieu environnemental (thermorésistance, résistance à certains agents chimiques et physiques...). Le passage de la forme sporulée à la forme végétative (phénomène de germination) est déclenché par la remise en conditions favorables de croissance, d'où cette étape de revivification.

La germination des spores peut être réalisée par toute méthode connue de l'état de la technique, par exemple, en chauffant la suspension des micro-organismes à 65°C pendant 20 minutes ou en ajustant le gradient osmotique des micro-organismes par diffusion séquentielle (séries d'extractions), diffusion progressive (dilution séquentielle) et par diffusion continue (dialyse). La méthode divulguée dans le brevet US 6,589,771 peut également être utilisée. L'utilisation de tampons ayant une osmolarité inférieure à celle des cellule, tel que le tampon PBS (Dubecco's phosphate Buffered Saline), pH 7.3, 0.9% NaCl est une autre méthode utitisable. L'addition de L-alanine dans le milieu de culture ayant un pH compris entre 5.5 et 7.0 à une température comprise entre 4° et 55°C peut aussi être utilisée pour la germination des spores, de même que le milieu connu sous le nom Brain Heart Infusion Broth, qui est un milieu plus particulièrement préféré pour la germination.

L'étape de revivification comprend la centrifugation de l'échantillon liquide pendant 1 à 3 minutes, de préférence 2 minutes à 10,000 x g, la resuspension du culot dans le milieu HIB et l'incubation sous agitation pendant une période d'environ 30 à 45, de préférence 40 minutes à une température comprise entre 4°C et 55°C de préférence 37° C.

Après cette étape de germination des spores, les micro-organismes sont soumis à la lyse et à l'étape d'extraction, pour l'extraction de l'ARN.

L'objectif de cette étape est de lyser l'ensemble des bactéries contenues dans l'échantillon concentré, après collecte, et d'en extraire la totalité des ARN dans un temps le plus court possible.

A cet égard, le protocole requiert que les micro-organismes soient lysés par voie chimique, mécanique ou thermique. Pour une lyse chimique, tout moyen de lyse approprié tel que la zymoliase, la cellulose, la β-glucururonidase, la lysostaphine, le lysozyme et d'autres peut être utilisé dans la méthode de l'invention.

Des moyens mécaniques pour la lyse des micro-organismes incluent l'homogénéisation, l'agitation en présence de billes de verre, la sonication, la presse de French, etc.. La lyse thermique consiste à chauffer les échantillons à une température de 80 à 90°C. Il est préférable d'utiliser une combinaison de lyse chimique utilisant un lysozyme et la lysostaphine suivi de la sonication à cette étape de la présente méthode.

Après lyse des cellules, l'ARN est extrait des cellules. Toute méthode connue de l'état de la technique peut être utilisée pour l'extraction de l'ARN des cellules, et notamment les nombreux kits d'extraction d'ARN disponibles dans le commerce. Par exemple, on utilisera le kit RNAeasy de QIAGEN^{®}, en suivant les instructions du fabriquant, ou tout autre kit d'extraction dont la mise en oeuvre est simple et rapide.

L'ARN obtenu peut être marqué radioactivement ou à l'aide d'un marqueur chimiluminescent. Alternativement, un ADN peut être synthétisé à partir d'un ARN en utilisant des méthodes de transcription inverse, ledit ADN étant marqué lors de l'étape de transcription inverse par incorporation de nucléotide modifié.

Cette étape de synthèse des sondes correspond à la transcription inverse (rétrotranscription) des ARN extraits en ADN complémentaire marqués. Deux différents types de marqueurs peuvent être utilisés en alternative dans le procédé de l'invention. Les acides nucléiques cibles peuvent être radiomarqués ou marqué à l'aide d'un marqueur permettant la révélation en mode chimiluminescent.

Tout marqueur radioactif peut être utilisé à cette fin, incluant ³²P, ³⁵S, ¹²⁵I, . P³³ H³, ¹³¹I, etc. Il est préférable d'utiliser le ³⁵S.

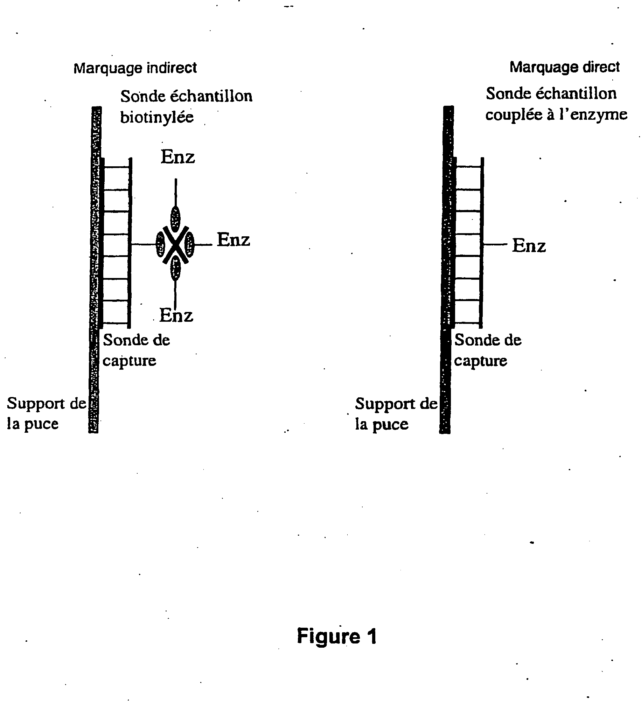

Le principe de la révélation chimiluminescente est basé sur l'utilisation d'enzymes qui dégradent spécifiquement des substrats chimiluminescents. Cette dégradation enzymatique provoque l'émission de photons. Dans ce cas, les sondes échantillons sont marquées soient directement avec l'enzyme, soient indirectement par un complexe moléculaire spécifique (complexe biotine / streptavidine / biotine-enzyme). Ces deux types de marquage des sondes échantillons sont représentés schématiquement dans la Figure 1.

Toute enzyme connue pour son utilisation dans la chimiluminescence peut être utilisée pour marquer l'acide nucléique cible de la présente invention. On citera, à titre d'exemples, la phosphatase alcaline, la β-galactosidase, la β-gluconidase, la β-glucosidase, la péroxidase, la luciférase etc. A titre d'exemples, les complexes de chimiluminescence comprennent la biotine, l'avidine et la streptavidine ou encore des complexes anticorps antibiotines ou anti-digoxygénine couplés à l'enzyme, fluorescéine-anticorps antifluorescéine.

En plus de l'échantillon marqué, on utilisera classiquement des témoins marqués également radioactivement ou à l'aide d'une marqueur chimiluminescent. Les ARN témoins sont caractérisés par l'absence totale d'hybridation sur les sondes de capture. Les témoins utilisés sont par exemple l'ARN codant la tyrosine hydroxylase (TH) ou l'ARN neo codant le gène de résistance à la néomycine.

Les avantages de la lecture en chimiluminescence sont notamment de permettre d'améliorer la sensibilité (possibilité d'amplifier le signal) et de réaliser une mesure quantitative. De plus, l'équipement nécessaire pour la mesure du signal est plus simple que pour les mesures en fluorescence (pas de source lumineuse excitatrice) et les manipulations sont facilitées par rapport à l'utilisation de produits radioactifs.

Le problème majeur de la révélation chimiluminescente réside dans le fait que les substrats chimiluminescents sont en solution liquide. Or, la diffusion des photons dans le liquide, ainsi que les mouvements du liquide sur la surface plane de la puce, provoquent une diminution significative de la résolution spatiale de lecture. Ce phénomène de diffusion de la lumière rend incompatible la mesure par rapport à la taille des spots sur les puces à ADN.

Un dispositif permettant d'immobiliser la phase liquide (substrat chimiluminescent) en contact avec la surface de la lame de verre, afin de limiter la diffusion de la lumière, a été défini.

Ce dispositif consiste en une membrane poreuse imprégnée du substrat. Cette membrane est déposée à la surface de la puce, créant ainsi un réseau tridimensionnel immobilisant la phase liquide. Ce système permet de réduire significativement les phénomènes de diffusion des photons, ceci rendant compatible la lecture avec le diamètre des spots.

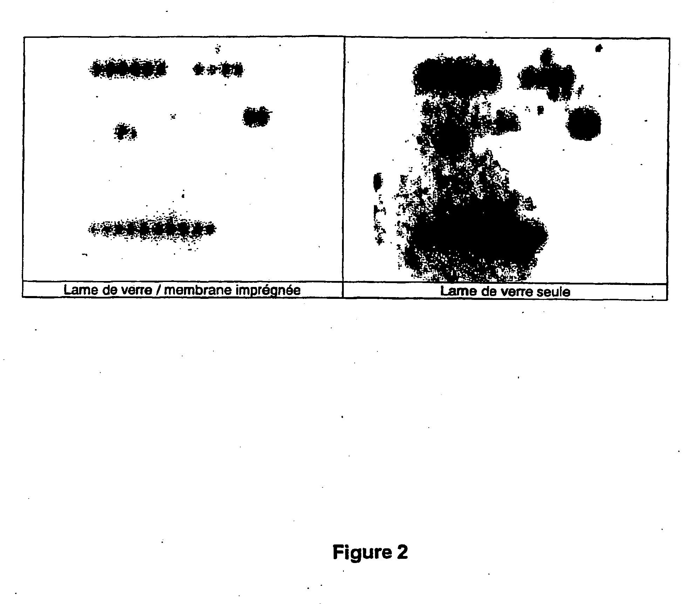

La Figure 2 représente les deux photographies de puces ci-dessous, qui permettent de comparer les résultats des mesures obtenues dans les configurations lame de verre / membrane imprégnée et lame de verre seule.

Avant de préparer les échantillons marqués de micro-organismes, on prépare les puces contenant les sondes de capture immobilisées. Les sondes de capture peuvent être des molécules d'ADN ou d'ARN.

Les sondes de capture sont de préférence choisies d'une part dans des régions spécifiques des ARNr16S de chaque agent, d'autre part dans des régions spécifiques de différents ARN messagers. Les sondes de capture correspondant aux ARNr16S ont été choisies dans les régions les plus variables de ces ARN, afin d'assurer l'identification des souches utilisées. Pour les sondes de capture correspondant à des ARNm, on choisira des ARNm dont le niveau d'expression est bien connu, afin de représenter au mieux les différents états physiologiques possibles pour les agents collectés. Ont été sélectionnés, des ARNm codant des protéines impliquées : dans la germination des spores; dans le cycle cellulaire; dans de grandes fonctions métaboliques; dans l'expression de la virulence. Avec ce type de choix large, il est raisonnable de pouvoir obtenir des signaux spécifiques quel que soit l'état physiologique de l'agent collecté, en sachant qu'une étape de revivification est effectuée avant la préparation de la sonde échantillon.

La taille des sondes de capture peut varier de 30 à 90 nucléotides, de préférence environ 70 nucléotides. Les sondes utilisables peuvent porter un groupement aminé à leur extrémité 5'.

Pour la fabrication des puces, une mince couche de verre est utilisée, recouverte de polylysine, de silane ou d'époxy. Dans un mode de réalisation préféré, l'époxy utilisé est le 3-glycidoxyl-propyl triméthoxy silane. Ce type d'époxy réagit spontanément en présence d'eau avec l'extrémité 5' aminée de la sonde de capture et permet une liaison covalente à l'extrémité 5' de la sonde. Cette liaison spécifique laisse la partie restante de la sonde libre pour une hybridation.

Les différents nucléotides qui sont aminés à l'extrémité 5' sont déposés sur la plaque de verre à l'aide d'un « spotter ». Par exemple, le diamètre moyen des spots peut être compris entre 10 et 150 µm. La distance entre les deux spots, d'un centre à l'autre peut être comprise entre 300 et 500 µm.

Les sondes échantillons (ADN ou ARN) préalablement marquées sont déposées sur la surface de la puce et vont s'hybrider par homologie de séquence au niveau des spots correspondants.

L'hybridation s'effectue en 30 minutes environ. Après cette phase d'hybridation, la puce est lavée et la lecture permet de révéler les hybridations effectives.

Le protocole pour l'étape d'hybridation est identique selon le type de marquage effectué, radioactif ou chimiluminescent.

Principalement, les sondes d'acides nucléiques sont d'abord dénaturées et un tampon d'hybridation est ajouté à l'acide nucléique dénaturé. Le mélange est ensuite placé sur la puce contenant les sondes de captures. La puce est ensuite placée dans une enceinte thermostatée à 42°C et laissée à incuber pendant environ 30 minutes. La puce est alors retirée de l'enceinte et lavée.

Selon le type de marquage, radioactif ou chimiluminescent, la méthode de révélation est différente. Si on utilise la radioactivité, la puce est couverte d'un scintillant solide et l'analyse est effectuée à l'aide d'un dispositif adapté (phosphorimager, film autoradio-graphique ...). Si un marqueur chimiluminescent est utilisé, une fine membrane de nylon est imprégnée d'un substrat chimiluminescent, qui est dilué dans un substrat approprié et placé sur la puce. La révélation se fait alors à l'aide du micro-imager.

### EXEMPLES

4 souches modèles représentant les bactéries gram - et gram + sous des formes végétatives ou sporulées ont été utilisées. Ces souches modèles correspondent à *Escherichia coli* (gram -), à *Pantoa agglomerans* (gram -, CIP 82 100), *à Bacillus thuringiensis,* sous espèce *kurstaki,* sous la forme végétative (gram +, CIP 105 674) et à *Bacillus globigii* sous la forme sporulée (CIP 77 18). Pour identifier ces quatre souches, nous avons tiré partie des données moléculaires disponibles dans les banques de données, en choisissant des sondes de capture immobilisées sur la puce correspondant à la fois aux régions variables des ARN ribosomaux 16S (ARNr16S) et des ARN messagers (ARNm) spécifiques. Dans un contexte plus large, la détection conjointe de ces deux classes d'ARN permet une identification, donnant ainsi accès au genre et à l'espèce.

### Exemple 1 : Préparation de l'échantillon

### A. Protocole de revivification.

Cette étape consiste à incuber les agents biologiques récupérés dans le milieu de collecte dans 200 µl de milieu de revivification.

Le milieu de collecte contenant les souches est centrifugé pendant 2 minutes à 10 000 x g.

Le culot bactérien est repris dans 200 µl de milieu de revivification HIB (Brain Heart Infusion Broth, ICN, Ref: 1002417), et mis à incuber sous agitation pendant 40 min à 37°C.

### B. Lyse et extraction

Les cultures sont centrifugées dans le milieu de revivification pendant 2 minutes à 10 000 x g.

Le culot bactérien est alors repris dans 50 µl de lysozyme à 12 mg/ml (solution diluée dans du TE) (Sigma), on y ajoute 50 µl de lysostaphine à 100 µg/ml (solution diluée dans du TE)(Sigma), et on effectue finalement la sonication des échantillons durant 30 secondes à 45 Hz.

L'incubation est ensuite réalisée pendant 10 minutes à 37°C, puis on y ajoute 350 µl de tampon RLT-2ME (kit RNAeasy de Qiagen), ainsi que 250µl d'éthanol absolu, que l'on mélange par aspiration / refoulement et que l'on dépose sur la colonne RNAeasy (colonne d'absorption). Le tout est alors centrifugé à 8000 x g pendant 15 sec.

La colonne est ensuite lavée avec 700 µl de tampon RW1 (centrifugation à 8000 x g pendant 15 sec), puis avec 500 µl de tampon RPE (deux fois) (centrifugation à 8000 x g pendant 15 sec).

La colonne est ensuite centrifugée pendant 2 minutes après le dernier lavage afin d'éliminer l'excédent de tampon de lavage.

20 µl d'eau stérile sont alors ajoutés au centre de la colonne et les ARN sont élués par centrifugation à 8000 x g pendant 1 min.

Si besoin, les ARN sont conservés à - 80°C.

### C. Synthèse des sondes échantillons

Deux protocoles différents sont possibles.

### Protocole pour la détection en mode radioactif

10 µL de pdN₆ (50 ng/µl) sont ajoutés à 7 µl du mélange d'ARN contenant les ARN totaux extraits précédemment, et 5 ng des ARN contrôles tyrosine hydroxylase (TH) et Néo.

Le mélange est alors incubé pendant 5 min à 70°C puis refroidi rapidement à 4°C.

Les réactifs suivants sont alors ajoutés à 4°C : 5 µl tampon RT 10 X, 10 µl MgCl₂ (25 mM), 5 µl DTT (100mM), 2.5 µl RNasin (40 U/µL), 1 µl mélange dCGT-TP (25 mM chacun), 5 µl dATP ³⁵S (1000Ci/mmol ; 10µCi/µL), 2.5 µl ddTTP (1 mM), 2 µl Super Script II (200 U/µl).

Le mélange doit ensuite incuber durant 2 min à 25°C, puis pendant 30 min à 42°C.

L'arrêt de la réaction et l'hydrolyse des ARN sont assurés par l'ajout de 7,5 µl de NaOH 2M (hydrolyse alcaline), une incubation pendant 5 min à 50°, l'ajout de 7,5 µl d'acide acétique 2,2 M (neutralisation) et enfin par la purification des sondes sur colonne d'exclusion P10 (Bio-Rad). Le volume final d'élution est de 40 µl.

### Protocole pour la détection en mode chimiluminescent

10 µL de pdN₆ (50 ng/µl) sont ajoutés à 7µl du mélange d'ARN contenant les ARN totaux extraits précédemment et 5 ng des ARN contrôles TH et Néo. Le mélange est alors incubé pendant 5 min à 70°C, puis refroidi rapidement à 4°C.

Toujours à 4°, les réactifs suivants sont ajoutés : 5 µl de tampon RT 10 X, 10 µl de MgCl₂ (25 mM), 5 µl de DTT (100mM), 2.5µl de RNasin (40 U/µl), 1 µl de mélange dCGA-TP (25 mM chacun), 5 µl de dUTP - biotine (1 mM), 1 µl de dTTP (10 mM), 1 µl de dATP ³⁵S (1000Ci/mmol ; 10µCi/µl), 2.5µl de ddTTP (1 mM) et 2 µl de Super Script II (200 U/µl).

Le mélange est alors incubé durant 2 min à 25°C, puis pendant 30 min à 42°C.

L'arrêt de la réaction et l'hydrolyse des ARN sont assurés par l'ajout de 7,5 µl de NaOH 2M (hydrolyse alcaline), une incubation pendant 5 min à 50°C et l'ajout de 7,5 µl d'acide acétique 2,2 M (neutralisation).

Les sondes sont ensuite purifiées sur colonne d'exclusion P10 (Bio-Rad) : le volume final d'élution est de 40 µl.

### Exemple 2 : Les sondes de capture

### A. Présentation des témoins

Comme témoin sur la puce, nous utilisons des sondes de capture correspondant aux ARN TH et néo. Ces ARN sont directement ajoutés aux ARN extraits juste avant la synthèse des sondes échantillons. Ils permettent ainsi, d'une part, de contrôler l'efficacité de la synthèse des ADNc marqués, d'autre part d'assurer les repérages lignes colonnes sur la puce.

### B. Choix des sondes de capture correspondant aux ARNr16S

Les séquences de ces sondes de capture sont les suivantes :

Pour les sondes de capture ARN16S, l'ensemble des hybridations croisées possibles d'un agent sur l'autre est parfaitement connu et peut être résumé dans le tableau de mésappariement ci-dessous.

| | **E.coli** | **Bth** | **Pagg** | **B. Globigii** |
|---|---|---|---|---|
| **16Scoli** | 0/70 | 31/70 | 13/70 | 38/70 |
| **16SBth1** | 40/70 | 0/70 | 35/70 | 16/70 |
| **16SBth2** | 32/70 | 0/70 | 28170 | 11/70 |
| **16Bth3** | 33/70 | 0/70 | 29170 | 11/70 |
| **16SPagg** | 11/70 | 33/70 | 0/70 | 39/70 |
| **16SBS1** | 38/70 | 15/70 | 36/70 | 0/70 |
| **16SBS2** | 30/70 | 10/70 | 33/70 | 0/70 |

### C. Choix des sondes de capture correspondant aux ARNm-

### Sondes de capture correspondant à des ARNm spécifiques de B. thuringiensis:

### 1. Gène cry spécifique de B. thuringiensis:

*cry* (accession number: AF278797) est un gène plasmidique exprimé seulement pendant la phase stationnaire du cycle de croissance cellulaire. Ce gène produit une endotoxine très efficace contre un grand nombre d'insectes.

### Oligonucléotides (70 mers) BTCY1

### 2. Gène inhA2 spécifique de B. thuringiensis:

Le gène *inhA2* (accession number: AF421888) est décrit dans l'article suivant : Fedhila, S., Nel, P. and Lereclus, D. 2002. The InhA2 metalloprotease of Bacillus thuringiensis strain 407 is required for pathogenicity in insects infected via the oral route. J. Bacteriol. 184 (12), 3296-3304.

Le gène *inhA* produit une metalloprotéase zinc dépendante. Cette protéine hydrolyse les cecropines et les attacines impliquées dans le système de défense humoral des insectes. L'expression de *inhA* commence au début de la sporulation et est indirectement activé par la protéine SpoA. Le gène *inhA2* produit une protéine qui a 66.2% d'identités avec la protéine InhA et qui contient un site de fixation du zinc. La protéine InhA2 joue un rôle important dans la virulence. Le gène *inhA2* est exprimé lorsque les bactéries sont en croissance ou sporulent dans un milieu riche (comme le milieu de revivification), alors que le gène *inhA* serait préférentiellement exprimé quand les cellules sont en milieu pauvre.

### Oligonucléotides (70 mers) BTinh1

### 3. Gène hblA spécifique de B. thuringiensis. B. cereus et Edwarsiella tarda:

Les gènes hblA (accession number AJ243149, AJ243147, AJ007794, AJ237785, AJ243163, L43071 )sont décrits dans les articles suivants :

Pruss,B.M., Dietrich,R., Nibler,B., Martlbauer,E. and Scherer,S. 1999. The hemolytic enterotoxin HBL is broadly distributed among species of the Bacillus cereus group. Appl. Environ. Microbiol. 65 (12), 5436-5442 (1999)

Okstad,O.A., Gominet,M., Purnelle,B., Rose,M., Lereclus,D. and Kolsto,A.B.1999. Sequence analysis of three Bacillus cereus loci carrying PlcR-regulated genes encoding degradative enzymes and enterotoxin. Microbiology 145 (Pt 11), 3129-3138.

Chen,J.D., Lai,S.Y. and Huang,S.L. 1996. Molecular cloning, characterization, and sequencing of the hemolysin gene from Edwardsiella tarda. Arch. Microbiol. 165 (1), 9-17

Le gène *hblA* produit une entérotoxine (hémolysine BL) qui se lie au complexe HBL.

### Oligonucléotides (70 mers)

### 4. Gène gyrB spécifique du groupe B. cereus:

Le gène *gyrB* (accession number:AF090331, AF136390, AF136387, AF090332, AF136388)est décrit dans l'article suivant :

Yamada,S., Ohashi,E., Agata,N. and Venkateswaran,K. 1999. Cloning and nucleotide sequence analysis of gyrB of Bacillus cereus, B. thuringiensis, B. mycoides, and B. anthracis and their application to the detection of B. cereus in rice. Appl. Environ. Microbiol. 65 (4), 1483-1490.

GyrB est une protéine de sous-unité B de l'ADN gyrase (topoisomérase type II). Elle permet la diminution des contraintes mécaniques affectant l'ADN double brins pendant les étapes de réplication et de transcription.

### Oligonucléotide (70 mers)

### D. Sondes de capture correspondant à des ARNm spécifiques de B. globigii:

### 1. gène recA spécifique du groupe B. cereus, B. anthracis et B. subtilis:

Le gène *recA* (accession number:AF229167, AY147925) est décrit dans les articles suivants : Ko,M., Choi,H. and Park,C. 2002. Group I self-splicing intron in the recA gene of Bacillus anthracis J. Bacteriol. 184 (14), 3917-3922; Maughan,H., Birky,C.W. Jr., Nicholson,W.L., Rosenzweig,W.D. and Vreeland,R.H. The paradox of the 'ancient' bacterium which contains 'modem' protein-coding gènes Unpublished.

RecA est une recombinase de type A, elle répare par recombinaison les erreurs de réplication de l'ADN.

Oligonucléotides RecA1 : spécifique du groupe *B. cereus* et *B. anthracis* (groupes proches de *B._thuringiensis) et* RecA2 : spécifique de *B. subtilis* dont *B. Globigii* est une sous espèce.

### Oligonucléotides

RecA1: spécifique du groupe *B*. *cereus* et *B. anthracis*

### RecA2: spécifique de B. subtilis

### 1. gène n17D spécifique de B.subtilis:

Le gène *n17D* (accession number:D31856)est décrit dans l'article suivant: Oda,M., Sugishita,A. and Furukawa,K. 1988. Cloning and nucleotide sequences of histidase and regulatory genes in the Bacillus subtilis hut operon and positive regulation of the operon. J. Bacteriol. 170 (7), 3199-3205.

Le gène *n17D* produit une enzyme, la 6-phospho-β-glucosidase impliqué dans le métabolisme du glucose.

### Oligonucléotide (70 mers) N17D

### 2. gène gpr spécifique de B.subtilis:

Le gène *gpr* (accession number Z99117) est décrit dans l'article suivant: Nessi, C., Jedrzejas, M.J., and Setlow, P.1998. Structure and mechanism of action of the protease that degrades small, acide-soluble spore proteins during germination of spores of Bacillus species. J. Bacteriol. 180 (19): 5077-5084.

Le gène gpr est spécifique de *B. subtilis.* Ce gène produit une protéase active pendant la germination des spores. Elle dégrade l'ADN associé à de petites protéines solubles (SASPs).

### Oligonucléotide (70 mers) gpr1

### 3. gène sleB spécifique de B.subtilis:

Le gène *sleB* (accession number: Z99117) est décrit dans l'article suivant : Moir, A., Corfe, B.M., and Behravan, J.2002. Spore germination. Cell. Mol. Life Sci.59: 403-409.

Le gène *sleB* est spécifique de *B. subtilis.* Ce gène est impliqué dans le stade précoce de la germination des spores. C'est une enzyme lytique qui dégrade le cortex des spores.

### Oligonucléotide (70 mers) sleB1

### 4. gène cw/D spécifique de B.subtilis:

Le gène *cwlD* (accession number: Z99117) est décrit dans l'article suivant : Moir, A., Corfe, B.M., and Behravan, J. 2002. Spore germination. Cell. Mol. Life Sci.59: 403-409. Le gène *cwlD* est spécifique de *B. subtilis.* Ce gène est impliqué dans le stade précoce de la germination des spores. C'est une hydrolase qui dégrade la paroi des spores. Son produit est la N-acetylmuramoyl-L-alanineamidase.

### Oligonucléotide (70 mers) cwlD1

### 5. gène cwlJ spécifique de B.subtilis:

Le gène cwlJ (accession number: Z99117) est décrit dans l'article suivant : Moir, A., Corfe, B.M., and Behravan, J. 2002. Spore germination. Cell. Mol. Life Sci. 59: 403-409.

Le gène cwlJ est spécifique de *B. subtilis.* Ce gène est impliqué dans le stade précoce de la germination des spores. C'est une hydrolase qui dégrade la paroi des spores.

### Oligonucléotide (70 mers) cwlJ1

### 6. gène dnaG spécifique de B. subtilis:

Le gène dnaG (accession number:Z99117) est décrit dans l'article suivant : Kunst,F., et al. 1997. The complete genome sequence of the gram-positive bacterium Bacillus subtilis Nature 390 (6657), 249-256.

Le gène dnaG code une primase intervenant au début de la réplication de l'ADN.

Oligonucléotide (70 mers) DnaGBS

### E. Sondes de capture correspondant à des ARNm spécifiques de P. agglomerans:

### 1. gène bg/A spécifique de P. agglomerans:

Le gène bglA (accession number:X79911 ) est décrit dans l'article suivant: Marri,L., Valentini,S. and Venditti,D. 1995. Cloning and nucleotide sequence of the bglA gene from Erwinia herbicola and expression of beta-glucosidase activity in Escherichia coli. FEMS Microbiol. Lett. 128 (2), 135-138.
BglA est une phospho-β-glucosidase A, elle hydrolyse la cellulose.

### Oligonucléotide (70 mers) BglA1

### 2. gène tutB spécifique de P. agglomerans:

Le gène *tutB* (accession number:AF418598, U25347) est décrit dans les articles suivants : Katayama,T., Suzuki,H., Koyanagi,T. and Kumagai,H. 2002. Functional analysis of the Erwinia herbicola tutB gene and its product. J. Bacteriol. 184 (11), 3135-3141. Foor,F. 1995. Nucleotide sequence and analysis of the tyrosine utilization genes of *Erwinia herbicola.* Unpublished

*tutB* est une tyrosine perméase (transporteur de la tyrosine) et participe au métabolisme de la tyrosine.

Oligonucléotide (70 mers) tut1

### 3. gène ipdC spécifique de P. agglomerans:

Le gène ipdC (accession number:L80006) est décrit dans l'article suivant :

Brandl, M.T., Quinones, B., and Lindow, S.E. 2001. Heterogeneous transcription of an indoleacetic acid biosynthetic gène in Erwinia herbicola on plant surfaces. PNAS 98(6): 3454-3459.

*ipdC* produit une enzyme l'indol-pyruvate décarboxylase impliquée dans le métabolisme d'une hormone d'origine végétale. L'expression du gène *ipdC* est induit par les plantes mais aussi par d'autres conditions environnementales comme des conditions de sécheresses.

### Oligonucléotide (70 mers) ipdc1

### 4. gène crtH spécifique de P. agglomerans:

Le gène *crtH* (accession number: L17139, M87280) est décrit dans l'article suivant :
Hundle,B., Alberti,M., Nievelstein,V., Beyer,P., Kleinig,H., Armstrong,G.A., Burke,D.H. and Hearst,J.E. 1994. Functional assignment of Erwinia herbicola Eho10 carotenoid genes expressed in Escherichia coli. Mol. Gen. Genet. 245 (4), 406-416.

Ce gène permet la synthèse du ß-carotène.

### Oligonucléotide crtH1

### F. Sondes de capture correspondant à des ARNm spécifiques de E. Coli :

### 1. gène pgm spécifique de E. coli:

Le gène pgm est décrit dans l'article suivant :
Welch,R.A., et al. 2002. Extensive Mosiac Structure Revealed by the Complete Genome Sequence of Uropathogenic Escherichia coli. PNAS 99 (26), 17020-17024.

Ce gène produit une enzyme qui participe à la fois à la dégradation et à la synthèse du glucose. Elle appartient à la famille des phosphohexose mutases.

### Oligonucléotide (70 mers) pgm1

### 2. gène lipB spécifique de E.coli:

Le gène *lipB* (accession number: AE016757.1|) est décrit dans l'article suivant: Sean W. Jordan and John E. Cronan, Jr. 2003. The Escherichia coli lipB Gene Encodes Lipoyl (Octanoyl)-Acyl Carrier Protein:Protein Transferase. J. Bacteriol. 185(5):1582-1589.

LipB est impliqué dans l'attachement du groupe lipoyle aux protéines en créant un pont amide. Cette protéine joint le groupe carboxyl de l'acide lipoïque aux groupes ε-aminés des résidus lysine.

### Oligonucléotide (70 mers) LipB1

### 3. gène dnaG spécifique de E.coli:

Le gène dnaG (accession number:V00274) est décrit dans l'article suivant : Smiley,B.L., et al.1982. Sequences of the Escherichia coli dnaG primase gene and regulation of its expression. PNAS. 79 (15), 4550-4554.

Le gène dnaG code une primase intervenant au début de la réplication de l'ADN.

### Oligonucléotide (70 mers) DnaG1

### Exemple 3- Fabrication des puces

Pour la fabrication des puces, nous utilisons des lames de verre époxy, QMT Epoxy slides (Quantifoil # S100005).Les lames époxy utilisées sont recouvertes de 3-glycidoxy-propyl trimethoxy silane.

Les différents oligonucléotides aminés en 5' (Eurogentec) sont dilués à 10 ou 15 µM dans la solution QMT spotting solution I recommandée par Quantifoil (QMT Epoxy Kit # S106010, Quantifoil). Les sondes de capture sont déposées sur les lames de verre à l'aide du « spotteur » QARRAY (Genetix) avec des aiguilles pleines (Proteigene). Le diamètre moyen des spots est de 200 µm et la distance inter-spots centre à centre est de 500 µm. Une fois le dépôt effectué, la liaison covalente entre la fonction aminée des sondes de capture et le support epoxy est réalisée en plaçant les lames dans une enceinte à atmosphère saturée en humidité pendant 30 min à 37°C. Les puces ainsi prêtes sont séchées à l'air et conservées à température ambiante à l'abri de l'humidité jusqu'à utilisation.

Juste avant de procéder à l'étape d'hybridation, les puces sont traitées suivant le protocole recommandé par Quantifoil : Elles doivent incuber 5 minutes dans la solution de lavage I à température ambiante, incuber 2 fois 2 minutes dans la solution de lavage II à température ambiante, incuber 10 minutes dans la solution de lavage III à température ambiante, incuber 1 minutes dans dH₂O à température ambiante, incuber 15 minutes dans 1X QMT Blocking solution à 50°C et enfin incuber 1 minutes dans H₂O à température ambiante.

### Présentation d'un plan de puce

La position des dépôts des sondes de capture sur la puce à ADN est présentée sur le schéma ci-dessous en Tableau 1

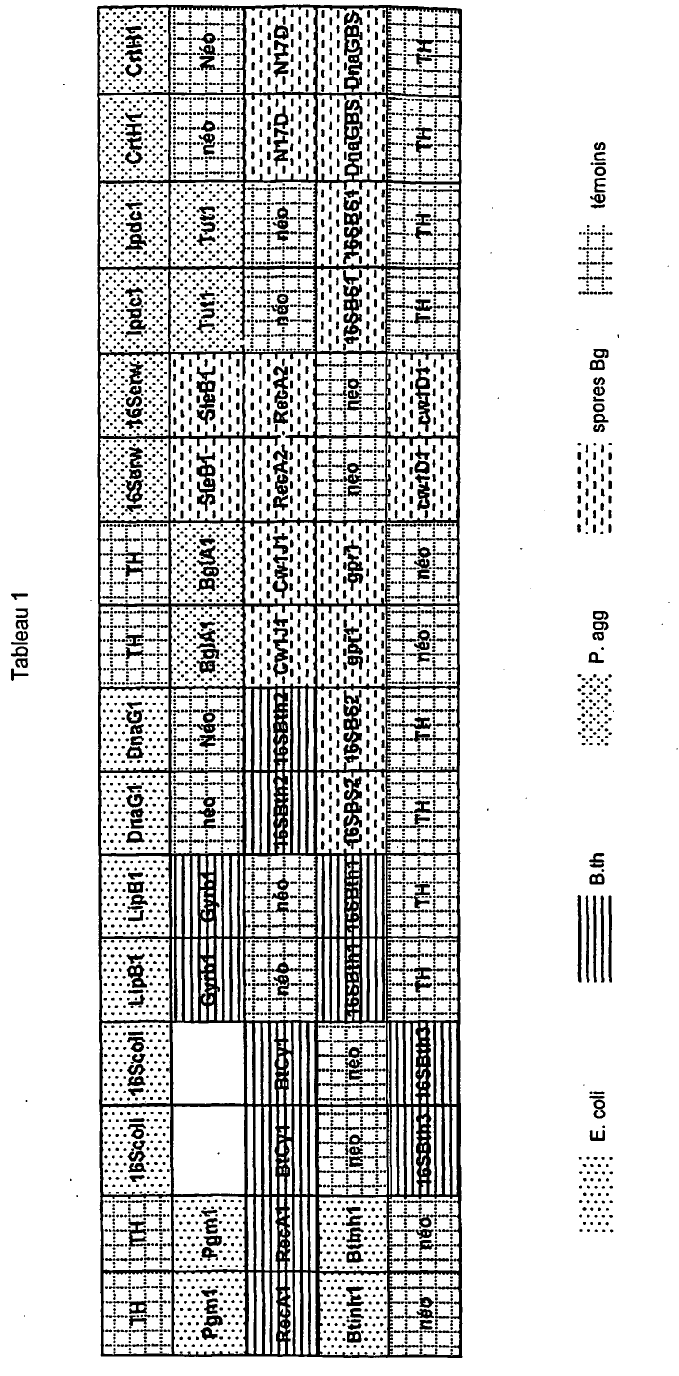

### Exemple 4 - Etape d'hybridation de la puce

Cette étape correspond à la fixation spécifique des sondes échantillons marquées sur les sondes de captures complémentaires (sur la puce). Elle consiste donc simplement à déposer sur la puce les sondes échantillons.

Suivant le mode de détection choisi (chimiluminescence ou radioactivité), deux protocoles différents sont possibles.

### A. Protocole pour la détection en mode radioactif

Les 40 µl de sonde échantillon marquée sont évaporés sous vide pour obtenir un volume final de 3,4 µl. La sonde est ensuite dénaturée pendant 2 min à 95°C, puis sont ajoutés sur la glace : 7 µl de SSC 20X filtré extemporanément, 20 µl de formamide 100%, 8 µl de dextran sulfate 25%, 0.6 µl de SDS 20% filtré, 1 µl d' ARNt levure à 3mg/ml.

La totalité du volume de cette solution est alors déposée sur la région de la puce contenant les spots et un morceau de parafilm placé sur le champ de puce pour éviter l'évaporation des sondes pendant l'hybridation.

La lame est placée dans la chambre d'hybridation dans des conditions garantissant l'absence d'évaporation de la sonde échantillon.

La chambre est ensuite placée dans une enceinte thermostatée à 42°C pendant 30 mn. La lame est alors sortie de la chambre, et lavée à 42°C suivant le protocole suivant : 2 min. dans la solution I : SSC 2X / SDS 0,1 %, 2 min. dans la solution II: SSC 1 X et 2 min. dans la solution III : SSC 0,2X.

La lame est finalement séchée par centrifugation durant 5 min. à 980 rpm, à température ambiante.

### B. Protocole pour la détection en mode chimiluminescent

Ce protocole est identique à celui présenté plus haut. Après le séchage de la lame par centrifugation durant 5 min à 980 rpm à température ambiante, les étapes suivantes sont ajoutées :

Les lames sont exposées à une source UV de 0,120 joules afin de réaliser des liaisons covalentes entre les sondes échantillons hybridées sur les sondes de capture.

Elles sont ensuite rincées rapidement dans du tampon PBS 1X, puis saturées avec 400µl de complexe ABC (Vector : Alkaline phosphates standard kit ABC ; réf. AK-5000) préparé 30 minutes à l'avance dans du tampon PBS 1X contenant 10 % de sérum de chèvre .

Les lames sont alors incubées 30 min sous atmosphère humide (assurée par un filtre imbibé de PBS au fond de la boîte dans laquelle est déposée la lame), à température ambiante, puis rincées une fois dans du PBS 1X à température ambiante (à l'obscurité).

Les lames sont ensuite lavées dans 3 bains de PBS 1X, 5. min chacun, sous agitation (à l'obscurité), et sont conservées si besoin à 4°C dans un bain de PBS 1X (à l'obscurité).

### Exemple 5 - Etape de lecture

### A. Sur les lames en mode radioactif

La lecture s'effectue avec le micro-imager (Biospace), la lame ayant été recouverte d'une feuille de scintillant solide.

### B. Sur les lames en mode chimiluminescent

Une membrane de nylon (Hybond N+) d'épaisseur 50 µm et d'aspect blanc, imprégnée de substrat CDPStar (Amersham) dilué 100 fois dans le tampon de dilution de substrat (Roche réf 1759 779), est ajouté sur la lame. La lecture s'effectue alors avec le micro-imager (Biospace).

### Exemple 6- Résultats

### A. Identification des quatre agents modèles

10⁶ cellules de chacun des quatre agents ont été traitées suivant le protocole présenté plus haut. Les sondes échantillons ont été préparées suivant le mode de marquage radioactif puis hybridées sur les puces à ADN. Les images ci-dessous correspondent à celles obtenues après 10 minutes d'acquisition sur le micro-imager.

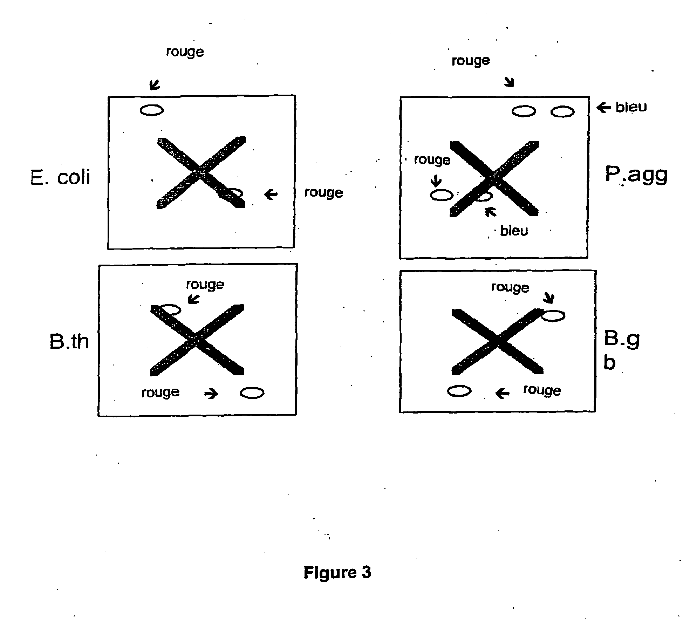

Les spots permettant à la Figure 3 d'identifier les quatre agents sont entourés en rouge pour les sondes de capture ARN16S et en bleu pour les ARNm.

Les tableaux récapitulatifs ci-dessous représentent le patron d'hybridation correspondant à chacun des agents modèles.

### E. coli + témoins TH et néo

**Tableau 2**

| | | | | | | | | | | | | | | | |
|---|---|---|---|---|---|---|---|---|---|---|---|---|---|---|---|
| TH | TH | 16Scoli | 16Scoli | | | | | TH | TH | 16SPa | 16SPa | | | | |
| | | | | | | Néo | néo | | | | | | | néo | néo |
| | | | | néo | néo | 16SBth2 | 16SBth2 | | | RecA2 | RecA2 | néo | néo | | |
| | | néo | néo | | | | | | | néo | néo | | | | |
| Néo | Néo | 16SBth3 | 16SBth3 | TH | TH | TH | TH | Néo | néo | | | TH | TH | TH | TH |

Les résultats montrent que les sondes échantillons d' *E. coli* s'hybrident spécifiquement avec les oligonucléotides des ARN16S d' *E*. *coli* et de *P*. *agglomerans.* Cependant, on remarque que l'intensité des spots est plus forte pour les ARN16S d' *E*. *coli* que pour les ARN16S de *P*. *agglomerans.* En effet le rapport moyen entre l'intensité des spots 16Scoli et celui des spots 16SPa est de 2.12.

Des hybridations non spécifiques sont observées comme pour l'oligonucléotide 16SBth3 16SBth2 spécifique de *B. thuringiensis* et l'oligonucléotide RecA2 spécifique de *B. globigii.* Cependant ces hybridations non spécifiques sont aussi observées pour tous les autres agents.

### P.agglomerans + témoins TH et néo

**Tableau 3**

| | | | | | | | | | | | | | | | |
|---|---|---|---|---|---|---|---|---|---|---|---|---|---|---|---|
| TH | TH | 16Scoli | 16Scoli | | | | | TH | TH | 16SPa | 16SPa | | | CrtH1 | CrtH1 |
| | | | | | | néo | néo | | | | | | | néo | néo |
| | | | | néo | néo | 16SBth2 | 16SBth2 | | | RecA2 | RecA2 | néo | néo | | |
| | | néo | néo | | | | | | | néo | néo | | | | |
| néo | néo | 16SBth3 | 16SBth3 | TH | TH | TH | TH | néo | néo | | | TH | TH | TH | TH |

Les résultats montrent que les sondes échantillons de *P. agglomerans* s'hybrident non seulement avec les ARN 16S de *P. agglomerans* et d' *E. coli* mais aussi avec les ARN16S de *B. thuringiensis,* 16SBth2 et 16SBth3. Le rapport moyen des intensités des spots entre 16SPa et 16Scoli est de 1,13 montrant que l'intensité des spots est plus intense pour les ARN16S de *P. agglomerans* que pour les ARN16S d' *E. coli.*

Un ARNm spécifique de *P. agglomerans* est détecté. Il s'agit de Crth1 qui code une protéine impliquée dans la bio-synthèse du β-carotène.

Des hybridations non spécifiques sont observées comme pour l'oligonucléotide 16SBth3 16SBth2 spécifique de *B. thuringiensis* et l'oligonucléotide RecA2 spécifique de *B. globigii.* Cependant ces hybridations non spécifiques sont aussi observées pour tous les autres agents.

### B.thuringiensis + témoins TH et néo

**Tableau 4**

| | | | | | | | | | | | | | | | |
|---|---|---|---|---|---|---|---|---|---|---|---|---|---|---|---|
| TH | TH | | | | | | | TH | TH | | | | | | |
| | | | | | | néo | néo | | | | | | | néo | néo |
| | | | | néo | néo | 16SBth2 | 16SBth2 | | | RecA2 | RecA2 | néo | néo | | |
| | | néo | néo | 16SBth1 | 16SBth1 | 16SBS2 | 16SBS2 | | | néo | néo | | | | |
| néo | néo | 16SBth3 | 16SBth3 | TH | TH | TH | TH | Néo | néo | | | TH | TH | TH | TH |

Les résultats montrent que les ARN16S spécifiques de *B. thuringiensis* sont visibles, 16SBth1, 16SBth2, 16SBth3. Les autres oligonucléotides non spécifiques ne sont pas révélés. L'oligonucléotide 16SBS2 spécifique de *B. globigii* est détecté en présence des sondes échantillons de *B. thuringiensis.* Ce résultat s'explique par la présence de 10 mésappariements entre l'ARN16S de *B. thuringiensis* et l'oligonucléotide 16SBS2 spécifique de *B. globigii.* Notre protocole d'hybridation ne permet pas de discriminer en dessous de 10 mésappariements.

Ici, seule la sonde de capture 16SBth1 permet d'identifier *B. thuringiensis.* En effet, les signaux correspondant à 16SBth2 et16SBth3 sont visibles pour tous les agents

### spores B.globigii + témoins TH et néo

**Tableau 5**

| | | | | | | | | | | | | | | | |
|---|---|---|---|---|---|---|---|---|---|---|---|---|---|---|---|
| TH | TH | | | | | | | TH | TH | | | | | | |
| | | | | | | néo | néo | | | | | | | néo | néo |
| | | | | néo | néo | 16SBth2 | 16SBth2 | | | RecA2 | RecA2 | néo | néo | | |
| | | néo | néo | | | 16SBS2 | 16SBS2 | | | néo | néo | 16SBS1 | 16SBS1 | | |
| néo | néo | 16SBth3 | 16SBth3 | TH | TH | TH | TH | néo | néo | | | TH | TH | TH | TH |

Les résultants montrent que les ARN 16S spécifiques de *B. globigii* sont visibles 16SBS1 et 16SBS2. Seule la sonde de capture 16SBS1 permet d'identifier *B. globigii.*

En résumé, les sondes spécifiques des ARN16S permettent de différencier tous les agents entre eux. La comparaison des valeurs d'intensité des signaux obtenus permet de discriminer entre *P. agglomerans* et *E. coli.* En effet, *E. coli* est identifié pour un rapport 16Scoli / 16SPa supérieur à 1 et *P. agglomerans* pour un rapoort inférieur à 1. De plus la détection de l'ARNm Crth, spécifique de *P. agglomerans* confirme l'analyse fondée sur la détection des ARN16S.

## Revendications

1. Méthode de détection rapide de micro-organismes dans un échantillon, comprenant :
a. l'extraction de l'ARN des micro-organismes présents dans ledit échantillon,
b. le marquage de l'ARN ou la rétrotranscription des ARN extraits en ADN complémentaire marqué,
c. l'hybridation de l'ADN complémentaire marqué ou de l'ARN marqué obtenu à l'étape (b) à l'aide de sondes de capture, de 30 à 90 mers, placées sur une puce et spécifiques de chaque micro-organisme, **caractérisée en ce que** l'hybridation est effectuée dans un temps compris entre 15 et 45 minutes, de préférence environ 30 minutes,
d. la détection de l'hybridation éventuelle, et,
e. l'analyse des résultats permettant de déterminer si les micro-organismes sont présents.

2. Méthode selon la revendication 1, **caractérisée en ce que** l'ADN ou l'ARN est marqué radioactivement.

3. Méthode selon la revendication 1, **caractérisée en ce que** l'ADN ou l'ARN est marqué au moyen d'un marqueur chimiluminescent.

4. Méthode selon la revendication 2, **caractérisée en ce que** le marqueur radioactif est le S³⁵.

5. Méthode selon la revendication 3, **caractérisée en ce que** le marqueur est un complexe enzymatique biotine/streptavidine/biotine.

6. Méthode selon la revendication 1, **caractérisée en ce que** avant l'étape (a), les micro-organismes sont soumis à une étape de revivification.

7. Méthode selon la revendication 6, **caractérisée en ce que** l'étape de revivification comprend la culture des microorganismes dans le milieu bouillon coeur-cervelle (brain heart infusion).

8. Méthode selon l'une des revendications 1 à 7, **caractérisée en ce que** l'ARN est extrait après une lyse des micro-organismes.

9. Méthode selon l'une quelconque des revendications 1 à 8, **caractérisée en ce que** la puce est une lame de verre recouverte de silane polylysine ou d'époxy.

10. Méthode selon la revendication 9, **caractérisée en ce que** l'époxy est constitué de 3-glycidoxy-propyl triméthoxy silane.

11. Méthode selon l'une quelconque des revendications 1 à 10, **caractérisée en ce que** la sonde de capture est d'une longueur d'environ 70 nucléotides.

12. Méthode selon l'une quelconque des revendications 1 à 11, **caractérisée en ce que** la sonde de capture porte un groupement amine à son extrémité 5'.

13. Méthode selon l'une quelconque des revendications 1 à 12, **caractérisée en ce que** les micro-organismes sont choisis dans le groupe suivant : *Staphylococcus aureus, Staphylococcus epidermidis, Staphylococcus saprophyticus, Micrococcus luteus, Micrococcus roseus, Lactococcus lactis, Streptococcus pneumoniae, Streptococcus bovis, Leuconostoc mesenteroides, Oenococcus oeni, Pediococcus damnosus, Enterococcus durans, Enterococcus faecalis, Enterococcus faecium, Neisseria gonorrhoeae, Neisseria meningitidis, Moraxella catarrhalis, Acinetobacter baumannii, Acinetobacter Iwoffii, Citrobacter diversus, Citrobacter freundii, Citrobacter diversus, Edwardsiella tarda, Enterobacter cloacae, Erwinia chrysantum, Erwinia carotovora, Escherichia coli, Hafnia alvei, Klebsiella oxytoca, Klebsiella pneumoniae, Morganella morganii, Pantoea agglomerans, Proteus mirabilis, Proteus vulgaris, Providencia alcalifaciens, Salmonella enterica subsp. enterica, Salmonella enterica, Salmonella paratyphi, Serratia marcescens, Shigella flexneri, Yersinia enterocolitica, Yersinia pestis, Vibrio parahaemolyticus, Vibrio cholerae, Aeromonas hydrophila, Pseudomonas aeruginosa, Pseudomonas fluorescens, Pseudomonas putida, Burkholderia cepacia, Burkholderia mallei, Burkholderia pseudomallei, Ralstonia pickettii, Alcaligenes faecalis, Acinetobacter baumannii, Acinetobacter Iwoffii, Stenotrophomonas maltophilia, Campylobacter jejuni, Bacillus cereus, Bacillus thuringiensis, Bacillus subtilis, Bacillus anthracis, Bacillus globigii, Listeria monocytogenes, Lactobacillus casei, Clostridium botulinum, Clostridium perfringens, Mycobacterium bovis BCG, Legionella pneumophila, Bordetella pertussis, Francisella tularensis, Brucella melitensis, Brucella abortus, Brucella suis, Brucella canis, Coxiella burnetti, Chlamydia pneumonia.*

14. Méthode selon la revendication 2 ou 4, **caractérisée en ce que** le temps de mise en oeuvre est d'environ 2½ heures.

15. Méthode selon la revendication 3 ou 5, **caractérisée en ce que** le temps de mise en oeuvre est d'environ 3 heures.

16. Méthode selon l'une quelconque des revendications précédentes, dans laquelle l'échantillon est un échantillon environnemental, tel que l'eau, les eaux usées, le sol, la boue, les déchets, les sédiments ou l'air.

17. Méthode selon la revendication 16, dans laquelle l'échantillon est un échantillon d'air obtenu à l'aide d'un échantillonneur d'air.

18. Méthode selon l'une quelconque des revendications 1 à 15, dans laquelle l'échantillon est un échantillon biologique tel que le liquide céphalo-rachidien, la lymphe, le liquide synovial, les larmes, l'urine, le sang, le plasma, le sérum, la peau, les tumeurs, les liquides amniotiques, des tissus, des cellules et des cultures cellulaires.

## Claims

1. A method for the rapid detection of microorganisms in a sample, comprising:
a) extracting the RNA from microorganisms present in a sample;
b) labeling the RNA or carrying our the retrotranscription of said extracted RNA into labeled complementary DNA;
c) hybridizing the labeled complementary DNA or labeled RNA obtained in step c) using capture probes, from 30 to 90 mers, placed on a chip and specific for each micro organism, **characterized in that** the hybridization is carried out in a time in the range 15 to 45 minutes, preferably about 30 minutes;
d) detecting any hybridization; and
e) analyzing the results to determine whether the microorganisms are present.

2. A method according to claim 1, **characterized in that** the DNA or RNA is radioactively labeled.

3. A method according to claim 1, **characterized in that** the DNA or RNA is labelled using a chemiluminescent marker.

4. A method according to claim 2, **characterized in that** the radioactive marker is ³⁵S.

5. A method according to claim 3, **characterized in that** said marker is a biotin/streptavidin/biotin enzymatic complex.

6. A method according to claim 1, **characterized in that** before step b), the microorganisms undergo a revivification step.

7. A method according to claim 6, **characterized in that** the revivification step comprises culturing microorganisms in brain heart infusion medium.

8. A method according to any one of claims 1 to 7, **characterized in that** the RNA is extracted after lysis of the microorganisms.

9. A method according to any one of claims 1 to 8, **characterized in that** the chip is a glass slide coated with polylysine silane or epoxy.

10. A method according to claim 9, **characterized in that** the epoxy is constituted by 3-glycidoxy-propyl trimethoxysilane.

11. A method according to any one of claims 1 to 10, **characterized in that** the capture probe is about 70 nucleotides long.

12. A method according to any one of claims 1 to 11, **characterized in that** said capture probe carries an aminated group at its 5' end.

13. A method according to any one of claims 1 to 12, **characterized in that** the microorganisms are selected from the following group:
*Staphylococcus aureus, Staphylococcus epidermidis, Staphylococcus saprophyticus, Micrococcus luteus, Micrococcus roseus, Lactococcus lactis, Streptococcus pneumoniae, Streptococcus bovis, Leuconostoc mesenteroides, Oenococcus oeni, Pediococcus damnosus, Enterococcus durans, Enterococcus faecalis, Enterococcus faecium, Neisseria gonorrhoeae, Neisseria meningitidis, Moraxella catarrhalis, Acinetobacter baumannii, Acinetobacter Iwoffii, Citrobacter diversus, Citrobacter freundii, Citrobacter diversus, Edwardsiella tarda, Enterobacter cloacae, Erwinia Chrysantum, Erwinia carotovora, Escherichia coli, Hafnia alvei, Klebsiella oxytoca, Klebsiella pneumoniae, Morganella morganii, Pantoea agglomerans, Proteus mirabilis, Proteus vulgaris, Providencia alcalifaciens, Salmonella enterica subsp enterica, Salmonella enterica, Salmonella paratyphi, Serratia marcescens, Shigella flexneri, Yersinia enterocolitica, Yersinia pestis, Vibrio parahaemolyticus, Vibrio cholerae, Aeromonas hydrophila, Pseudomonas aeruginosa, Pseudomonas fluorescens, Pseudomonas putida, Burkholderia cepacia, Burkholderia mallei, Burkholderia pseudomallei, Ralstonia pickettii, Alcaligenes faecalis, Acinetobacter baumannii, Acinetobacter lwoffii, Stenotrophomonas maltophilia, Campylobacter jejuni, Bacillus cereus, Bacillus thuringiensis, Bacillus subtilis, Bacillus anthracis, Bacillus globigii, Listeria monocytogenes, Lactobacillus casei, Clostridium botulinum, Clostridium perfringens, Mycobacterium bovis BCG, Legionella pneumophila, Bordetella pertussis, Francisella tularensis, Brucella melitensis, Brucella abortus, Brucela suis, Brucella canis, Coxiella burnetti, Chlamydia pneumonia.*

14. A method according to claim 2 or claim 4, **characterized in that** the implementation time is about 2 ½ hours.

15. A method according to claim 3 or claim 5, **characterized in that** the implementation time is about 3 hours.

16. A method according to any one of the preceding claims, wherein said sample is an environmental sample, such as water, waste water, earth, mud, waste, sediment or air.

17. A method according to claim 16, **characterized in that** the microorganisms are collected using an air sampler.

18. A method according to any one of claims 1 to 15, wherein said sample is a biological sample, such as cephalorachidian liquid, lymph, synovial liquid, tears, urine, blood, plasma, serum, skin, tumors, amniotic fluid, tissues, cells and cell cultures.

## Patentansprüche

1. Verfahren zum schnellen Nachweis von Mikroorganismen in einer Probe, umfassend :
a. die Extraktion der RNA der Mikroorganismen, die in der Probe vorhanden sind,
b. die Markierung der RNA oder die reverse Transkription der extrahierten RNAs in markierte komplementäre DNA,
c. die Hybridisierung der markierten komplementären DNA oder der markierten RNA, die in Schritt (b) erhalten worden ist, mit Hilfe von Einfang-Sonden von 30- bis 90-meren, die auf einem Chip angeordnet und für jeden Mikroorganismus spezifisch sind, **dadurch gekennzeichnet, dass** die Hybridisierung in einer Zeitspanne zwischen 15 und 45 min, vorzugsweise etwa 30 min ausgeführt wird,
d. den Nachweis der etwaigen Hybridisierung und
e. die Analyse der Ergebnisse, welche erlaubt, zu bestimmen, ob die Mikroorganismen vorhanden sind.

2. Verfahren nach Anspruch 1, **dadurch gekennzeichnet, dass** die DNA oder die RNA radioaktiv markiert wird.

3. Verfahren nach Anspruch 1, **dadurch gekennzeichnet, dass** die DNA oder die RNA mittels eines chemolumineszierenden Markers markiert wird.

4. Verfahren nach Anspruch 2, **dadurch gekennzeichnet, dass** der radioaktive Marker ³⁵S ist.

5. Verfahren nach Anspruch 3, **dadurch gekennzeichnet, dass** der Marker ein enzymatischer Biotin/Streptavidin/Biotin-Komplex ist.

6. Verfahren nach Anspruch 1, **dadurch gekennzeichnet, dass** vor dem Schritt (a) die Mikroorganismen einem Wiederbelebungsschritt unterworfen werden.

7. Verfahren nach Anspruch 6, **dadurch gekennzeichnet, dass** der Wiederbelebungsschritt die Kultivierung der Mikroorganismen in Herz-Hirn-Brühenmedium ("brain heart infusion") umfasst.

8. Verfahren nach einem der Ansprüche 1 bis 7, **dadurch gekennzeichnet, dass** die RNA nach einer Lyse der Mikroorganismen extrahiert wird.

9. Verfahren nach einem der Ansprüche 1 bis 8, **dadurch gekennzeichnet, dass** der Chip ein mit Polylysinsilan oder Epoxy-Material bedecktes Glasplättchen ist.

10. Verfahren nach Anspruch 9, **dadurch gekennzeichnet, dass** das Epoxy-material aus 3-Glycidoxypropyltrimethoxysilan gebildet wird.

11. Verfahren nach einem der Ansprüche 1 bis 10, **dadurch gekennzeichnet, dass** die Einfang-Sonde eine Länge von etwa 70 Nukleotiden aufweist.

12. Verfahren nach einem der Ansprüche 1 bis 11, **dadurch gekennzeichnet, dass** die Einfang-Sonde an ihrem 5'-Ende eine Amingruppe trägt.

13. Verfahren nach einem der Ansprüche 1 bis 12, **dadurch gekennzeichnet, dass** die Mikroorganismen in der folgenden Gruppe ausgewählt werden: *Staphylococcus aureus, Staphylococcus epidermidis, Staphylococcus saprophyticus, Micrococcus luteus, Micrococcus roseus, Lactococcus lactis, Streptococcus pneumoniae, Streptococcus bovis, Leuconostoc mesenteroides, Oenococcus oeni, Pediococcus damnosus, Enterococcus durans, Enterococcus faecalis, Enterococcus faecium, Neisseria gonorrhoeae, Neisseria meningitidis, Moraxella catarrhalis, Acinetobacter baumannii, Acinetobacter Iwoffii, Citrobacter diversus, Citrobacter freundii, Citrobacter diversus, Edwardsiella tarda, Enterobacter cloacae, Erwinia chrysantum, Erwinia carotovora, Escherichia coli, Hafnia alvei, Klebsiella oxytoca, Klebsiella pneumoniae, Morganella morganii, Pantoea agglomerans, Proteus mirabilis, Proteus vulgaris, Providencia alcalifaciens, Salmonella enterica subsp. enterica, Salmonella enterica, Salmonella paratyphi, Serratia marcescens, Shigella flexneri, Yersinia enterocolitica, Yersinia pestis, Vibrio parahaemolyticus, Vibrio cholerae, Aeromonas hydrophila, Pseudomonas aeruginosa, Pseudomonas fluorescens, Pseudomonas putida, Burkholderia cepacia, Burkholderia mallei, Burkholderia pseudomallei, Ralstonia pickettii, Alcaligenes faecalis, Acinetobacter baumannii, Acinetobacter Iwoffii, Stenotrophomonas maltophilia, Campylobacter jejuni, Bacillus cereus, Bacillus thuringiensis, Bacillus subtilis, Bacillus anthracis, Bacillus globigii, Listeria monocytogenes, Lactobacillus casei, Clostridium botulinum, Clostridium perfringens, Mycobacterium bovis BCG, Legionella pneumophila, Bordetella pertussis, Francisella tularensis, Brucella melitensis, Brucella abortus, Brucella suis, Brucella canis, Coxiella burnetti, Chlamydia pneumonia.*

14. Verfahren nach Anspruch 2 oder 4, **dadurch gekennzeichnet, dass** die Ausführungszeit etwa 2 ½ h beträgt.

15. Verfahren nach Anspruch 3 oder 5, **dadurch gekennzeichnet, dass** die Ausführungszeit etwa 3 h beträgt.

16. Verfahren nach einem der vorangegangenen Ansprüche, in welchem die Probe eine aus der Umwelt entnommene Probe, wie Wasser, Abwasser, Boden, Schlamm, Abfälle, Sedimente oder Luft, ist.

17. Verfahren nach Anspruch 16, in welchem die Probe eine Luftprobe ist, die mit Hilfe eines Luft-Probennehmers erhalten worden ist.

18. Verfahren nach einem der Ansprüche 1 bis 15, in welchem die Probe eine biologische Probe, wie Liquor cerebrospinalis, Lymphe, Synovia, Tränen, Urin, Blut, Plasma, Serum, Haut, Tumore, Fruchtwasser, Gewebe, Zellen und Zellkulturen, ist.


### LISTAGE DES SEQUENCES

<110> BERTIN TECHNOLOGIES
   CENTRE NATIONAL DE LA RECHERCHE SCIENTIFIQUE
<120> METHODE DE DETECTION RAPIDE DE MICRO-ORGANISMES SUR PUCES A ADN
<130> B5746b-JAZ/LV/SDU(ext PCT)
<140> PCT/FRXX/XXXXX
   <141> 2004-10-08
<150> FR 0311921
   <151> 2003-10-10
<160> 28
<170> PatentIn version 3.1
<210> 1
   <211> 70
   <212> DNA
   <213> Homo sapiens
<400> 1
   gggacatgta cccatgttgg ctgaccgcac atttgcccag ttctcccagg acattggact      60
   tgcatctctg                                                             70
<210> 2
   <211> 70
   <212> DNA
   <213> artificial: neomycine
<400> 2
   aggatctcct gtcatctcac cttgctcctg ccgagaaagt atccatcatg gctgatgcaa      60
   tgcggcggct                                                             70
<210> 3
   <211> 72
   <212> DNA
   <213> Escherichia coli
<400> 3
   gtactttcag cggggaggaa gggagtaaag ttaatacctt tgctcattga cgttacccgc 60
   agaagaagca cc 72
<210> 4
   <211> 70
   <212> DNA
   <213> Pantoa agglomerans
<400> 4
   aaagtacttt cagcgggagg aggcgatggg ttaataacct gtcgattgac gttaccgcag 60
   aagaagcacc 70
<210> 5
   <211> 70
   <212> DNA
   <213> Bacillus thuringiensis
<400> 5
   cattttgaac tgcatggttc gaaattgaaa ggcggcttcg gctgtcactt atggatggac 60
   ccgcgtcgca 70
<210> 6
   <211> 70
   <212> DNA
   <213> Bacillus thuringiensis
<400> 6
   gataactccg ggaaaccggg gctaataccg gataacattt tgaactgcat ggttcgaaat 60
   tgaaaggcgg 70
<210> 7
   <211> 70
   <212> DNA
   <213> Bacillus thuringiensis
<400> 7
   cgggaaaccg gggctaatac cggataacat tttgaactgc atggttcgaa attgaaaggc 60
   ggcttcggct 70
<210> 8
   <211> 70
   <212> DNA
   <213> Bacillus globigii
<400> 8
   taataccgga tggttgtttg aaccgcatgg ttcaaacata aaaggtggct tcggctacca 60
   cttcacgatg 70
<210> 9
   <211> 70
   <212> DNA
   <213> Bacillus globigii
<400> 9
   tgaaggtttt cggatcgtaa agctctgttg ttagggaaga acaagtgccg ttcgaatagg 60
   gcggtacctt 70
<210> 10
   <211> 70
   <212> DNA
   <213> Bacillus thuringiensis
<400> 10
   cgcttgaaag ttgggttgga aatcgtaaga acacaagggc taggagtgtt gtcaagagcc 60
   aatatatcgc 70
<210> 11
   <211> 70
   <212> DNA
   <213> Bacillus thuringiensis
<400> 11
   gcaagttcca acttcaaaag caaagcaagc tccatataaa ggatctgttc gatcggataa 60
   agtattagtg 70
<210> 12
   <211> 70
   <212> DNA
   <213> Bacillus thuringiensis
<400> 12
   ggatacgaaa gtaaaaaaac agcttttaga tacattgaat ggtattgttg aatacgatac 60
   aacatttgac 70
<210> 13
   <211> 71
   <212> DNA
   <213> Bacillus anthracis
<400> 13
   cgtaatatta aattaacaat tgaagataaa cgtgaacata agcaaaagaa agagttccac 60
   tatgaaggtg g 71
<210> 14
   <211> 70
   <212> DNA
   <213> Bacillus anthracis
<400> 14
   ggtggtcgtg cgttgaaatt ctattcaact gttcgtcttg aagtgcgtcg tgcggagcaa 60
   ttaaaacaag 70
<210> 15
   <211> 70
   <212> DNA
   <213> Bacillus subtilis
<400> 15
   cgacgtgaag cagaatgtgc ttggtgtcaa gaaggaaaca ttagatcgtt ttggggcggt 60
   cagcaaggag 70
<210> 16
   <211> 70
   <212> DNA
   <213> Bacillus subtilis
<400> 16
   cgcaaaacac ttttcttctc agatgttcag gcaagaggcg catacccggg atatatgaaa 60
   cgctatctgg 70
<210> 17
   <211> 70
   <212> DNA
   <213> Bacillus subtilis
<400> 17
   ggcacttgtt taagcttcag ccggaaaacg tacaggaggg ttacaggcct gtcagtgctt 60
   ttgcaccggg 70
<210> 18
   <211> 70
   <212> DNA
   <213> Bacillus subtilis
<400> 18
   gggaatacat tcacgcatta cggaaaaatt ccgctaaagt atcagacgaa accatcaaaa 60
   gcagcaacac 70
<210> 19
   <211> 70
   <212> DNA
   <213> Bacillus subtilis
<400> 19
   ggctatagcc gacgaaaagc tgaggatcta agacaacgag tcaaattaat aaaccattca 60
   gaggcggagc 70
<210> 20
   <211> 70
   <212> DNA
   <213> Bacillus subtilis
<400> 20
   cgcgtttgag gctgtgactc atggatattt ttatcaaagg gcgcgagata gcgagcgtgc 60
   ccttgcacgc 70
<210> 21
   <211> 70
   <212> DNA
   <213> Bacillus subtilis
<400> 21
   ggcccgcctt catatcagaa agcaggaaag agcagtctta tttgaagggt ttgctgatgt 60
   ctatacggcc 70
<210> 22
   <211> 70
   <212> DNA
   <213> Pantoa agglomerans
<400> 22
   ggttgaccag tttgtactga ttaatgaacc aaccgttgaa gttgcaacaa aaatcatggc 60
   cgagaaacga 70
<210> 23
   <211> 70
   <212> DNA
   <213> Pantoa agglomerans
<400> 23
   gctaccttct ctggcctgat atggcatgtc gaaggcgcta aacttattga cagtgccgcc 60
   tgggcgctgc 70
<210> 24
   <211> 70
   <212> DNA
   <213> Pantoa agglomerans
<400> 24
   cgatctgcag cccttcagtg ccagcgtggg taatgaacgt tttgcgccgt tgtcgatggc 60
   ggatgcgctc 70
<210> 25
   <211> 70
   <212> DNA
   <213> Pantoa agglomerans
<400> 25
   cccgcgcaag ggcgtatttg agctaaacga tctctttgcg gtggtgtttg ccggggtggc 60
   tatcgcgctg 70
<210> 26
   <211> 70
   <212> DNA
   <213> Escherichia coli
<400> 26
   ggcggttccg gtatcgaata ctggaagcgt attggcgagt attacaacct caacctgact 60
   atcgttaacg 70
<210> 27
   <211> 70
   <212> DNA
   <213> Escherichia coli
<400> 27
   ggggcaacag gtgatgtatg tgttgcttaa cctgaaacgc cgtaaactcg gtgtgcgtga 60
   actggtgacc 70
<210> 28
   <211> 70
   <212> DNA
   <213> Escherichia coli
<400> 28
   cgggtgattg gttttggcgg gcgcgtgctg ggcaacgata cccccaaata cctgaactcg 60
   ccggaaacag 70